(19) 
Europäisches
Patentamt
European
Patent Office
Office européen
des brevets

(11) **EP 2 167 192 B1**

(12) **EUROPEAN PATENT SPECIFICATION**

(45) Date of publication and mention
of the grant of the patent:
**07.11.2018 Bulletin 2018/45**

(51) Int Cl.:
***A61N 5/06*** *(2006.01)*

(21) Application number: **08774070.0**

(22) Date of filing: **11.06.2008**

(86) International application number:
**PCT/EP2008/057333**

(87) International publication number:
**WO 2008/152076 (18.12.2008 Gazette 2008/51)**

(54) **SYSTEM FOR OPTICAL TOMOGRAPHY FEEDBACK CONTROL OF DOSIMETRY FOR PHOTODYNAMIC THERAPY**

SYSTEM ZUR REGELUNG DER DOSIEMETRIE MITTELS OPTISCHER TOMOGRAFIE FÜR PHOTODYNAMISCHE THERAPIE

SYSTÈME POUR COMMANDE DE RÉTROACTION DE TOMOGRAPHIE OPTIQUE DE DOSIMÉTRIE POUR UNE THÉRAPIE PHOTODYNAMIQUE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **11.06.2007 US 943235 P**

(43) Date of publication of application:
**31.03.2010 Bulletin 2010/13**

(60) Divisional application:
**12160652.9**

(73) Proprietor: **SpectraCure AB**
**226 43 Lund (SE)**

(72) Inventors:
- **SWARTLING, Johannes**
**S-226 51 Lund (SE)**
- **AXELSSON, Johan**
**S-224 60 Lund (SE)**

(74) Representative: **KIPA AB**
**P O Box 1065**
**251 10 Helsingborg (SE)**

(56) References cited:
**EP-A- 1 470 837        WO-A-03/041575**
**WO-A-2008/020050    US-A- 6 138 046**
**US-B1- 6 549 284**

- **ALTSCHULER MARTIN ET AL: "Optimized interstitial PDT prostate treatment planning with the Cimmino feasibility algorithm" MEDICAL PHYSICS, AIP, MELVILLE, NY, US, vol. 32, no. 12, 9 November 2005 (2005-11-09), pages 3524-3536, XP012075215 ISSN: 0094-2405**
- **WEERSINK R A ET AL: "Techniques for delivery and monitoring of TOOKAD(WST09)-mediated photodynamic therapy of the prostate: clinical experience and practicalities" PROCEEDINGS OF THE SPIE - THE INTERNATIONAL SOCIETY FOR OPTICAL ENGINEERING SPIE-INT. SOC. OPT. ENG USA, vol. 5689, no. 1, 2005, pages 112-122, XP007906809 ISSN: 0277-786X**
- **ANDERSSON-ENGELS S ET AL: "Integrated system for interstitial photodynamic therapy" PROCEEDINGS OF THE SPIE - THE INTERNATIONAL SOCIETY FOR OPTICAL ENGINEERING SPIE-INT. SOC. OPT. ENG USA, vol. 5123, 2003, pages 293-302, XP007906808 ISSN: 0277-786X**
- **JOHANSSON A ET AL: "System for integrated interstitial photodynamic therapy and dosimetric monitoring" PROCEEDINGS OF THE SPIE - THE INTERNATIONAL SOCIETY FOR OPTICAL ENGINEERING SPIE-INT. SOC. OPT. ENG USA, vol. 5689, no. 1, 2005, pages 130-140, XP007906796 ISSN: 0277-786X**

EP 2 167 192 B1

**(Cont. next page)**

- JOHANSSON A ET AL: "In vivo measurement of parameters of dosimetric importance during interstitial photodynamic therapy of thick skin tumors" JOURNAL OF BIOMEDICAL OPTICS SPIE USA, vol. 11, no. 3, May 2006 (2006-05), pages 34029-1, XP007906795 ISSN: 1083-3668
- SVENSSON T ET AL: "In vivo optical characterization of human prostate tissue using near-infrared time-resolved spectroscopy" JOURNAL OF BIOMEDICAL OPTICS SPIE USA, vol. 12, no. 1, January 2007 (2007-01), pages 14022-1, XP007906794 ISSN: 1083-3668
- JOHANSSON ANN ET AL: "Realtime light dosimetry software tools for interstitial photodynamic therapy of the human prostate" MEDICAL PHYSICS, AIP, MELVILLE, NY, US, vol. 34, no. 11, 19 October 2007 (2007-10-19), pages 4309-4321, XP012103210 ISSN: 0094-2405

**Description**

**Field of the Invention**

This invention pertains in general to the field of photodynamic light therapy (PDT). More particularly the invention relates to a system, for internally controlling and adjusting therapy parameters in such a PDT system by a control of light dosimetry. More particularly, the invention refers to interstitial tumor PDT.

**Background of the Invention**

Photodynamic therapy (PDT) is a cancer treatment modality that has shown promising results in terms of selectivity and efficacy; see e.g. Dougherty TJ, et. al.: Photodynamic therapy, Journal of the National Cancer Institute 1998; 90: 889-905.

Photodynamic therapy (PDT) has become a clinically more accepted method for treating certain types of malignancies in various organs, partly due to advantages such as the possibility of repeated treatment and restriction of the treatment-induced tissue damage to irradiated sites. Tissue damage depends on the total light dose, the tissue oxygenation and the sensitizer concentration.

The deposited light dose throughout the tissue is affected by the photosensitizer concentration. In addition the photosensitizer bleaching has shown correlation with PDT effect. A faster bleaching rate suggests a higher level of tissue damage hence the photobleaching rate could be used as an implicit dose metric during the treatment. It is clear that monitoring the sensitizer fluorescence is indeed important.

In interstitial photodynamic therapy (IPDT) one aims to induce tissue damage in a tissue volume using interstitially placed optical light sources, such as optical fibers.

Significant inter- and intra-patient variations in the absorption and scattering coefficients of prostate tissue to be treated have been measured utilizing spatially resolved spectroscopy. In addition, any treatment-induced variations in absorption and scattering, possibly due to changes in blood content and tissue oxygenation status, directly influence the light distribution during the treatment; see e.g. A. Johansson et. al., Journal of Biomedical Optics, 11(3), 2006. Clearly, there is a need to monitor the tissue optical properties in individual patients both before and during the treatment.

An issue is PDT's inadequate capability to resolve inter- and intra-variation in the tissue to be treated and surrounding tissue. Thus patient safety may be improved by specifically treating desired tissue. Furthermore, there is a need to shorten treatment time, with maintained treatment result.

Hence, an improved control of dosimetry for PDT would be advantageous and in particular allowing for increased flexibility, cost-effectiveness, and/or patient safety would be advantageous.

**Summary of the Invention**

Accordingly, embodiments of the present invention preferably seeks to mitigate, alleviate or eliminate one or more deficiencies, disadvantages or issues in the art, such as the above-identified, singly or in any combination by providing a system according to the appended patent claims.

According to the invention, a Photo Dynamic Therapy (PDT) system is provided. The system comprises a control unit, a dosimetry unit and an optical diagnostic tomographic calculation unit. The control unit is adapted to control PDT therapy in said dosimetry unit based on input data from said optical diagnostic tomographic calculation unit.

Also disclosed is a method of controlling a photodynamic therapy (PDT) treatment, which comprises performing measurements of tissue in or at a subject for said PDT treatment based on at least one light source, before and/or during said PDT treatment, and using results of said measurements for tomographic reconstruction of therapy parameters for a feed-back to control and/or optimize said PDT treatment.

Also disclosed is a computer program for performing the previous method, storable on a computer readable medium, and adapted to be executed by a processing device, which comprises a code segment for using results of measurements for tomographic reconstruction of therapy parameters for a feed-back to control and/or optimize PDT treatment.

Furthermore, a use of optical tomographic data in Photo Dynamic Therapy (PDT) system is disclosed for a feed-back to control and/or optimize PDT treatment of the Photo Dynamic Therapy (PDT) system. Embodiments of the invention are defined in the dependent claims.

Some embodiments of the invention provide for shorter treatment time.

Some embodiments of the invention also provide for improved patient safety.

It should be emphasized that the term "comprises/comprising" when used in this specification is taken to specify the presence of stated features, integers, steps or components but does not preclude the presence or addition of one or more other features, integers, steps, components or groups thereof.

**Brief description of drawings**

In the following description reference is being made to the accompanying drawings, in which

Figure 1 is a schematic flow chart with a diagnostic tomographic calculation unit incorporated within a light dosimetry unit.

Figure 2 is a schematic drawing illustrating a prostate model retrieved from ultra sound slices.

Figure 3 is a schematic drawing (left) showing a reconstructed absorption in the same cross-section for three simulated levels of absorption coefficient, and a graph (right) showing the same reconstructions extracted for each source fiber.

Figure 4 is a graph showing averaged reconstructed absorption coefficient for the different evaluation schemes, wherein error bars define $\pm 1$ standard deviation of the absorption coefficient.

**Detailed description**

The following description focuses on a PDT system and method, and in particular to an interstitial PDT system and method, with reference to an example of a practical treatment of prostate cancer. However, it will be appreciated that the treatment is not limited to this application but may be applied to PDT or IPDT treatment of many other organs, including for example liver, oesophagus, pancreas, breast, brain, lung, trachea, eye, urinary tract, brain stem, spinal marrow, bone marrow, kidneys, stomach, intestines, pancreas, gall bladder, etc as well as superficial organs including the skin.

A PDT system is for instance disclosed in WO 2003/041575 and WO2008/020050, both from the same applicant. However, the PDT system disclosed in WO 2003/041575 may be further improved e.g. with regard to intra- and inter-patient variations.

A method is provided, for controlling a photodynamic therapy (PDT) treatment for which pre-treatment measurements of tissue subject is performed based on at least one light source, before and/or during the PDT treatment. The results of the measurements are used for optical tomographic reconstruction of therapy parameters for a feed-back to control and/or optimize an on-going PDT treatment in real-time or intermediate.

An apparatus for optical diagnostic tomography is provided. The apparatus is adapted to obtain the spatial distribution of tissue chromofores within a tissue to be treated.

A computer program for processing by a computer is provided. The computer program comprises inter-linked code segments for control of PDT therapy, i.e. dosimetry. Code segments specially adopted for diagnostic tomographic calculations resolve spatial distributions of tissue chromophores from measurements. A specially adopted code segment in the control unit within the dosimetry, makes use of the results from the diagnostic tomographic code segments for controlling and/or optimizing dosimetry for the PDT treatment.

The properties of light (e.g., wavelength distribution, intensity, spatial distribution, angular distribution, phase properties) are altered by interactions with the chemical and structural properties of the tissue through which the light has propagated. These alterations provide information about the tissue properties which may be used for optimization of PDT.

Generally most today clinical used PDT-system comprise a dosimetry unit not fully equipped to consider intra- and inter-variations e.g. in the prostate gland. That means these systems adopt a homogenous properties approach for the control algorithm in respect to the affected tissue in and around the prostate gland.

This disclosure adopts a scheme aiming to assess the spatial distribution of e.g. the photosensitizer during an IPDT-treatment of e.g. the human prostate based on spatially resolved measurements inside the prostate. Furthermore, the optical tomography may additionally be based on direct reconstruction of tissue chromophores such as hemoglobin, oxyhemoglobin, water, fat and photosensitizer. The scheme solves the inverse problem using the diffusion equation. Fundamentally for the disclosure is light propagation modeling using a finite element method. Furthermore, control of interstitial photodynamic therapy (PDT) is provided by means of modulation control and/or optical tomography. Measurements are provided for feed-back to control and optimize PDT treatment. The measurements are performed before and/or during the treatment.

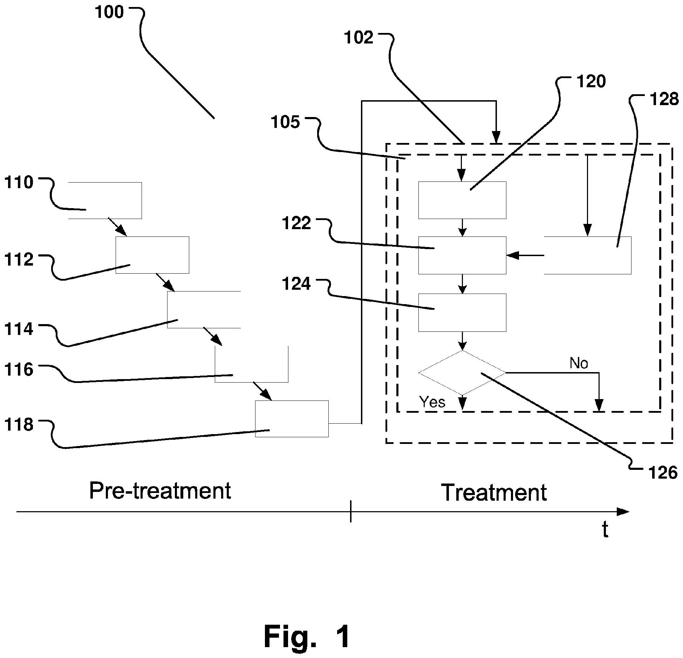

Figure 1 shows a general IPDT treatment scheme. Prior to the prostate IPDT treatment a transrectal ultra-sound investigation is performed to assess the geometry of the target tissue as well as nearby organs at risk (OAR) 110. Cross-sectional slices are retrieved of the prostate geometry and adjacent tissue types. The slices form the basis for a three dimensional rendering of the tissue volume where the extent of the prostatic gland, urethra, rectum, upper and lower sphincters and the cavernous nerve bundles are delineated by the urologist, 112. Based on the 3D model of the geometry a random-search algorithm provides positions for the optical fibers, 114. The optical fibers are then positioned at these positions, based on that virtual planning.

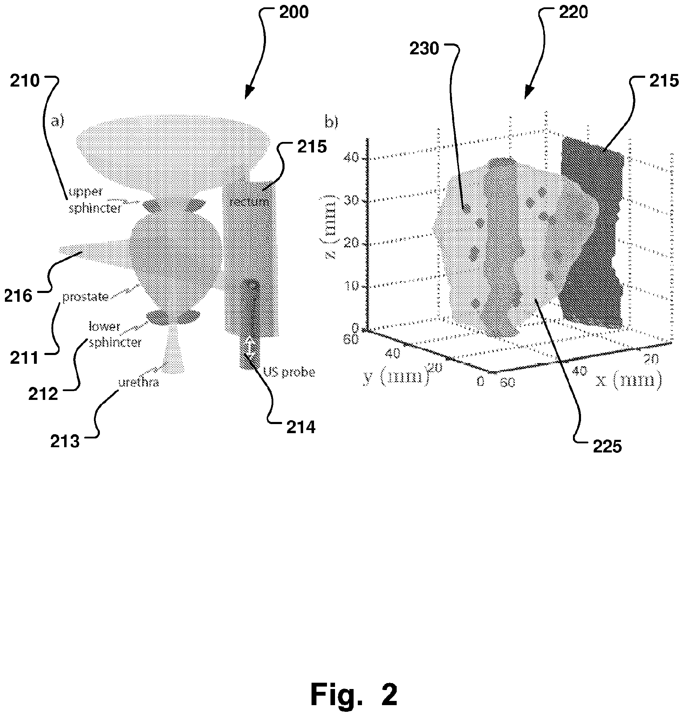

The light transmission signals for the therapeutic light is modeled using e.g. finite element method, FEM. A realistic model is e.g.retrieved from an ultra-sound examination prior to a e.g. brachytherapy therapy session. Using a set of ultra-sound slices, 216 a 3D-model of the prostate and surrounding organs are created, see figure 2. The organs delineated in the model shown are normal tissue, prostate 211, urethra 213 and rectum 215. The urethra may be simulated to be filled with air hence low absorption and scattering will be assigned to this tissue region.

Utilizing e.g. hollow steel needles, the optical fibers, also referred to as treatment fibers, are guided into position, 116. Within this fourth step, the urologist is given the opportunity to update the final fiber positions as these might deviate slightly from the set of positions calculated by the random-search optimization algorithm. Information on the geometry and the actual fiber positions is used as input for an optimization algorithm to predict required irradiation times for all source fibers, 118. This inverse problem utilizes optimization algorithm, e.g. based on Block-Cimmino, where the fiber-specific light irradiation times are computed in order to maximize the delivered treatment parameters, e.g. the fluence rate in the prostate, while sparing sensitive organs.

Figure 2 illustrates a sample three-dimensional geometry model 220, with 1 mm voxel side lengths, including the target tissue 225, i.e. the prostate 211, the OAR, consisting of the urethra 213, rectum 215, and normal, surrounding tissue as well as the source fiber positions 230. This geometry, representing the "test" geometry used in an example, was created based on eight ultrasound images from a patient with a glandular volume of approximately 27 cm3 and treatment fiber positions were calculated by the algorithm, in the step 114 of figure 1.

Following the pre-treatment planning, the IPDT session commences. The IPDT instrumentation, 102, comprises a dosimetry unit 105, to control and monitor delivered light dose. In some examples this unit is configured to monitor and control the on-going IPDT session in real-time.

In some examples the unit is configured to intermittent monitor and control the status of the IPDT session. For these later examples the light irradiation will halt and spatially resolved measurements are performed.

Typically said dosimetry unit involves iterating measurement sequence unit, 120. Immediately following a measurement sequence an evaluation of the measurement data to assess the effective attenuation coefficient within volumetric subsets of the prostate gland, is performed in control unit 122. An optimization algorithm, e.g. a Block-Cimmino algorithm, 124, is then executed in order to update the fiber irradiation times.

The control unit is supplied with additional input data from a diagnostic tomographic calculation unit, 128. Optical tomography is used as an input for controlling dosimetry in the PDT system. Alternatively, optical diagnostic tomographic calculation unit 128 may be provided external to a dosimetry unit 105 and provide data to the later. In addition, or alternatively output data from diagnostic tomographic calculation unit 128 may be provided for feed-back in other means. For instance such data may be provided as input data to measurement sequence unit 120. From measurement sequence unit 120 the tomographic data may be further provided to control unit 122. Also, the tomographic output data may be provided for other types of access to control unit 122. For instance, the data may be stored on a data carrier or in a memory unit (not shown).

After each measurement session the possible change in absorption coefficient for the therapeutic light is evaluated. Executing, e.g. a Cimmino algorithm will update the treatment times whenever an absorption change has occurred. Steps (120) to (128) are iterated until the remaining treatment time as predicted by the Block-Cimmino algorithm equals zero, 126. When this limit is reached the dosimetry unit 105 declare end treatment session to the PDT instrumentation 102. The implemented scheme, where 122, 124 and 128 constitute the real-time dosimetry module, is also referred to as Interactive Dosimetry by Sequential Evaluation (IDOSE).

Tools for tomographic reconstruction

Within the optical diagnostic tomographic calculation unit, 128, several tools may be adopted to reconstruct and resolve tomographic information from obtained parameter measurements.

A measurement sequence involves monitoring of the light transmission between the treatment fibers. Each optical fiber is sequentially emitting laser light while the neighboring optical fibers detect the transmitted light as well as the fluorescence induced by the laser light. The amount of neighboring optical fibers involved for detection may be based on several parameters, e.g. the necessary discretization needed, available calculation power to mention a few. In the following description six neighboring optical fibers are used as an example. Using a diffusion approximation for the light propagation the fluorescence light can be described using a steady-state coupled diffusion equation.

$$\nabla\left[D_x(r)\nabla\Phi_x(r)\right]-\mu_{ax}\Phi_x(r)+S_x(r)=0 \qquad (1)$$

$$\nabla\left[D_m(r)\nabla\Phi_m(r)\right]-\mu_{am}\Phi_m(r)+\eta\mu_{af}(r)\Phi_x(r)=0 \qquad (2)$$

Here subscript x denotes excitation photons and m denotes fluorescence photons. $S_x$ is the source term. The diffusion coefficient is defined by $D_{x,m} = [3(\mu_{ax,m} + \mu_{sx,m})]^{-1}$. The coupling is governed by the photosensitizer fluorescence yield $(\eta\mu_{af})$ where $\mu_{af}$ is the absorption coefficient and is the quantum yield of the photosensitizer. Further the absorption coefficient is connected to the concentration by $\mu_{af} = \varepsilon \cdot c$ where $\varepsilon$ is the extinction coefficient and c is the concentration.

**Reconstruction of photosensitizer concentration**

The inverse problem of finding the photosensitizer concentration relies on minimization of

$$\chi^2 = \sum_{i=1}^{NM} \left( \Phi_{m_i}^{meas} - \Phi_{m_i}^{calc} \right)^2 + \alpha \sum_{j=1}^{NN} \left( L\left( \mu_{af_j} - \mu_{af_0} \right) \right)^2 \tag{3}$$

Here NM denotes number of measurements and NN number of nodes. L is a matrix defined by $L_{i,j}=1$ when i=j, $L_{i,j}=-1/NV$ when i and j are in the same tissue region with NV voxels and zero otherwise. The matrix L is built based on the transrectal ultrasound slices. The minimization is performed in an iterative procedure where the fluorescence emission $\left( \Phi_{m_i}^{calc} \right)$ at all detectors is calculated and the photosensitizer absorption coefficient is updated, in each iteration, e.g. using the generalized Moore-Penrose inverse.

$$\Delta\mu_{af} = \left[ J^T J + \beta L^T L \right]^{-1} J^T \left( \Phi_m^{meas} - \Phi_m^{calc} \right) \tag{4}$$

In the iterative procedure the Jacobian in Eq. (4) is calculated using the finite element method. The reconstruction mesh is constructed by a coarse cube grid of e.g. 15x15x15 voxels. The iteration was stopped when the projection error in Eq. (3) was lower than 1%.

**Reconstruction of absorption**

The IPDT-instrument performs transmission monitoring using steady-state measurements. In a monitoring session, performed when the therapeutic light is off, each treatment fiber sequentially emit light. The transmitted fluence rate is collected by the six neighboring fibers. The arrangement of the fibers governs minimal probing through urethra. Assuming that the reduced scattering is known the absorption coefficient can be assessed through various evaluation schemes.
In some examples the diagnostic tomographic calculation unit, 128, is based on a linear algorithm. In addition, in some embodiments the diagnostic tomographic calculation unit is based on Diffuse Optical Tomography (DOT).
The first scheme, i.e. the linear algorithm, is to approximate the tissue as homogeneous and infinite. This provides the possibility to use the Green solution to the diffusion equation, stated in Eq. (1).

$$\phi(r_s, r_d) = \frac{P}{4\pi D |r_s - r_d|} \exp\left( -\sqrt{\frac{\mu_a}{D}} |r_s - r_d| \right) \tag{1}$$

Here P is the laser power [W] emitted at the fiber tip, $D = 1/(3(\mu_a + \mu_s'))$ [mm] is the diffusion coefficient whereas $r_s$ and $r_d$ are the source and detector position respectively. Rearranging Eq. (1) yields a linear relation where $\mu_{eff} = \sqrt{\mu_a / D}$ is the slope, see Eq. (2).

$$\ln\left( \phi(r_s, r_d) \cdot |r_s - r_d| \right) = \ln\left( \frac{P}{4\pi D} \right) - \mu_{eff} |r_s - r_d| \tag{2}$$

Since the scattering is assumed to be constant throughout the geometry $\mu_a$ can be calculated. The linear fit is performed one time for each treatment fiber rendering fiber specific absorption.
The preferred implementation is the more rigorous approach using DOT. Here the change between two states is analyzed using a perturbative approach. This approach relies on the fact that the change in absorption in a small volume element inside the geometry will affect the detected intensity. To what extent each source-detector pair is affected is described by the sensitivity matrix, W, stated in Eq. (3),

$$\ln\left(\Phi_m\right) - \ln\left(\Phi_0\right) = W\Delta\mu_{am} = -\frac{\phi(r_s, r_k)\phi(r_k, r_d)}{\phi(r_s, r_d)}\left(\mu_{am} - \mu_{a0}\right) \qquad (3)$$

Here $\Phi_m$ defines the measurement m while $\Phi_0$ is the initial state measurement. Further $r_s$ and $r_d$ are, as before, the source and detector positions while $r_k$ is the position of voxel k in the geometry. In an example the prostate geometry was discretized into 4096 elements. Utilizing all source-detector pairs within a measurement sequence the absorption change from the initial state, i.e. $\mu_{am}$ can be retrieved using Tikhonov regularization. The matrix equation to solve for is given in Eq. (4).

$$\Delta\ln\left(\Phi_m\right) = W\Delta\mu_{am} \qquad (4)$$

where $\Delta\ln(\Phi_m)$ is a 108x1 vector holding the difference of the measured quantities. In this specific setting each of the 18 optical fibers with the 6 neighboring optical fibers arrange in a vector. W is a matrix of size 108x4096 and $\Delta\mu_{am}$ is a 4096x1 vector of unknown absorption differences between the two states. $\Delta\mu_{am}$ is retrieved through regularized matrix inversion, Eq. (5).

$$\Delta\mu_{am} = \left(W'W + \lambda L'L\right)^{-1}W'\Delta\ln\Phi_m \qquad (5)$$

The $\lambda$-term is a regularization parameter. In this example we use 1% of the maximum diagonal element of W'W. The matrix L is adopted from Brooksby et. al. Journal of Biomedical Optics, 10(5), 2005. L governs spatial a priori information about the tissue geometry. L is effectively a laplacian filter smoothing the solution in all voxels belonging to the same tissue type. The priori-matrix holds information about what voxels within the geometry that belong to the same tissue type and the construction is defined below.

$$L_{i,i} = 1$$

$$L_{i,j} = -\frac{1}{N_{region}},$$

$$L_{i,j} = 0,$$

if voxel i and j are in the same tissue region, then $N_{region}$ is the number of voxels otherwise

The problem at hand is to reconstruct the absorption change for the temporally increasing absorption within the prostate. Two approaches to the DOT scheme may be applied. Some examples may adopt scheme using a linear fit to the simulated measurements at the first monitoring sequence to initiate the initial state (Linear+DOT). Some examples may use a homogeneous model as the initial state which adopts optical properties from mean prostate optical properties of small patient population (Green+DOT). The optical properties may be assessed by using time resolved spectroscopy. The default optical properties were assessed to be $\mu_a$ =0.05 1/mm and $\mu_s$'=0.87 1/mm.

The above specification describes optical tomography of the prostate for PDT dosimetry based on the diffusion equation of radiative transfer (Eq. 1) and linearised reconstruction using the Rytov approximation (Eqs. 3 and 4).

In some implementations of PDT online dosimetry the emitted light used for the measurements is steady-state, i.e., it has a constant intensity over the duration of the measurement. In a signal processing interpretation, this corresponds to measuring only the response of the tissue at zero frequency. However, a frequency response of the tissue at higher frequencies may carry additional information. The typical bandwidth needed for this kind of measurement is approximately, but not limited to, in the region of 100 kHz - 10 GHz.

In some examples the diagnostic tomographic calculation unit is based on obtained measurements from modulated light sources, which gives more information about the tissue than steady-state light emission gives.

The detected signal may either be recorded in the frequency domain (FD) or in the time domain (TD). FD data is represented by intensity as a function of frequency (power spectrum) and phase as a function of frequency. TD data is represented by intensity as a function of time. FD and TD representation are mathematically equivalent, linked by the Fourier transform.

By means of a model for light propagation in tissues, it is possible to extract unique optical characteristics of the tissue from the measurement data. In general, the optical characteristics constitute properties related to the absorption and scattering of light in the tissue, which is the information needed for accurate light dosimetry during interstitial PDT.

Since the FD or TD data carries more information of the absorption and scattering properties of the tissue than conventional steady-state data, it has the potential to yield more accurate estimates of the true optical properties of the tissue. For example, steady state-measurements cannot easily discriminate between, on the one hand, local bleeding close to light sources or detectors, and, on the other hand, increased absorption in the tissue volume as a whole. With FD or TD data such discrimination is possible.

Another advantage of using modulated measurements lies in the fact that the use of FD or TD data is the basis of powerful methods for diffuse optical tomography, wherein the aim is to make a reconstruction of the three-dimensional distribution of optical properties of the tissue volume. Steady-state data may be used for tomographic reconstruction, but the use of FD or TD data expands the mathematical possibilities and thus the potential for accurate reconstruction of optical properties in the tissue.

Three schemes to retrieve temporal changes of the absorption coefficient have been presented here. Using simulated data as input for a DOT reconstruction algorithm the absorption coefficient increase could be estimated within 10% from the true value whereas a spatially resolved linear regression scheme showed larger deviations.

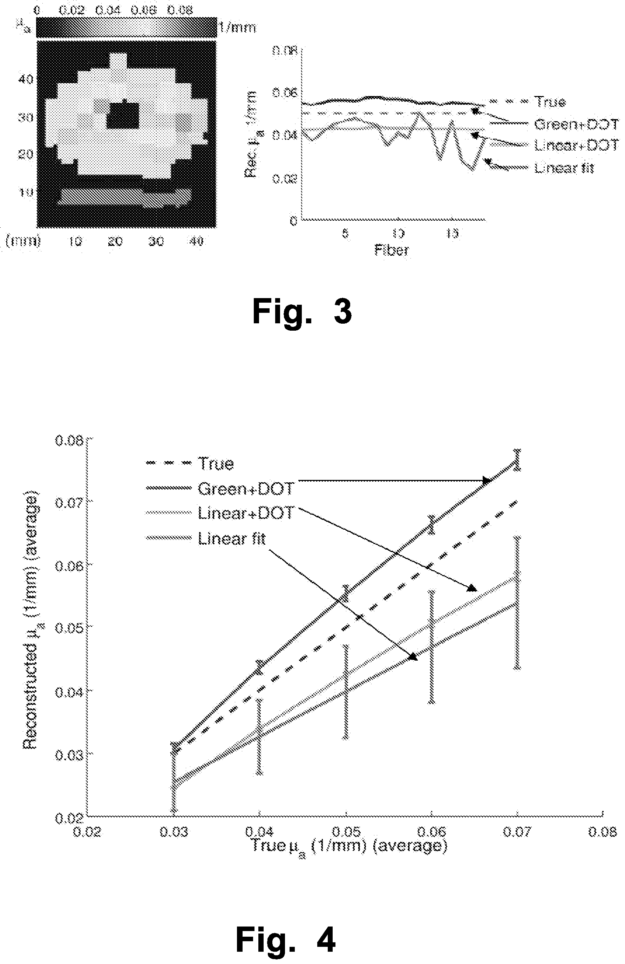

It is clearly shown in contrast to a linear approach resolving spatial absorption in the prostate gland using a tomographic approach estimates the values with less variability. In figure 4 shown the error bars, reflecting one standard deviation, are narrower using DOT than corresponding linear approach. Thus, accurate reconstruction of optical properties in tissue treated by the PDT is provided.

The Green-DOT scheme constantly overestimates the absorption coefficient in this particular case. This effect is due to the assumption, in the initial state, that the tissue is homogeneous with the default optical properties. The difference between two states is not only due to an absorption increase in the prostate but is also affected by the reduced scattering which in the true prostate model is lower for tissue types other than the prostate. This fact renders larger overestimation errors for higher values of absorption, as seen in Figure 4.

The Linear-DOT and the Linear regression schemes both underestimates the reconstructed absorption as compared to the true absorption in the prostate. In the case of Linear-DOT this is due to the initial state where the optical properties were retrieved from $\mu_{eff}$ through a linear fit. Since the surrounding tissue types has lower values of both $\mu_a$ and $\mu_s'$ the initial state will be underestimated and hence affect the consecutive reconstructions. The effective attenuation is shown as a function of $\mu_a$ and $\mu_s'$ in Figure 4.

In Figure 3 (left) one cross-section at z=22 mm, is shown for each simulated monitoring sequence. The false color coding represent reconstructed absorption coefficient. The spatial a priori information clearly smoothes the solution although artifacts are seen close to urethra and at the source fiber positions.

To ease the comparison with the linear regression approach the absorption coefficient from the three-dimensional reconstruction was extracted for each fiber. Here the average of all voxels within a sphere, of 20 mm radius, surrounding the fiber position was calculated. The absorption coefficient is shown for each fiber in Figure 3 (right). Comparing the linear regression results and the two DOT-schemes it is clearly visible that fibers close to urethra and rectum (fiber 14 and 17) render large errors for the linear fit whereas the spatial prior constrain the DOT reconstructed absorption to be more homogeneous for the fibers. Further the average of the reconstructed absorption coefficient of prostate tissue was calculated and the comparison for all simulated monitoring sequences is shown in Figure 3.

The described method is not limited to PDT of the prostate but is applicable to PDT dosimetry of any organ.

Other methods for optical tomography are also possible to be used for implementation, including:

- Forward models based on general solutions to the transport equation of radiative transfer (Boltzmann equation).
- Linearised reconstruction using the Born approximation or higher order approximations.
- Reconstruction methods based on time-domain or frequency-domain measurements.
- Non-linear iterative reconstruction methods based on either the Rytov or Born approximations or higher order approximations.
- Direct reconstruction of tissue chromophores such as hemoglobin, oxyhemoglobin, water, fat and photosensitizer.

**Example**

FEM was used to model the fluence rate distribution within a model representing the prostate, urethra, rectum and sphincters, Fig. 2a, acquired during a transrectal ultrasound investigation. Two simulation runs were performed in a cube mesh containing all tissue types. Approximately 18000 nodes were used in the mesh and the bulk optical properties were assumed constant, i.e. $\mu_{ax}$=0.67 cm$^{-1}$, $\mu_{am}$=0.33 cm$^{-1}$ and $\mu_{sx}$'=8.2 cm$^{-1}$, $\mu_{sm}$'=7.4 cm$^{-1}$ for excitation and emission wavelengths respectively. 1% normal distributed noise was added to the optical properties. The optical properties were well within the range relevant optical properties for the human prostate.

The first simulation run (*homogeneous bleaching*) aimed to investigate the possibility to track a homogeneous photosensitizer bleaching. The mTHPC concentration was set to be the same for all voxels within the prostate and sequentially decreased between simulations. The target photosensitizer concentrations were 0.5, 0.4, 0.3, 0.2 and 0.1 $\mu$M.

In the second simulation run (*heterogeneous bleaching*) the prostate was split in two regions. The voxels within each half were set to hold different mTHPC concentrations. This simulation was performed for three levels on mTHPC concentrations, i.e. 0.5 and 0.3 $\mu$M, 0.3 and 0.15 $\mu$M as well as 0.25 and 0.05 $\mu$M for the first and the second half respectively.

For the homogeneous bleaching simulations it is seen that the scheme can track the change of the simulated concentration. Small heterogeneities are also seen in the reconstructed results. These are due to the interstitially placed simulated optical fibers. Since the sensitivity is very high close to a source or detector this phenomenon is inherent in the reconstruction scheme. The spatial prior, formed using the ultra-sound slices, smoothes the solution within the prostate.

The reconstructed results when the prostate holds two different regions with different amount of mTHPC reveals that the scheme can track heterogeneous changes in the probed tissue volume.

Different method steps than those described above, performing the method by hardware or software, may be provided. The different features and steps may be combined in other combinations than those described.

## Claims

1.  A Photo Dynamic Therapy (PDT) system comprising at least one light source, optical fibers for interstitially positioning in tissue, and a dosimetry unit (105), for carrying out tomography during said PDT; wherein said dosimetry unit (105) comprises a measurement sequence unit (120) for monitoring parameter measurements of light transmission between said fibers when sequentially emitting light while neighboring optical fibers are used to detect transmitted light as well as fluorescence light, a control unit (122) and an optical diagnostic tomographic calculation unit (128), wherein said optical diagnostic tomographic calculation unit (128) is adapted for tomographic reconstruction of a spatial distribution of therapy parameters including photosensitizer concentration, which is derived from said fluorescence light, and optical properties including absorption coefficient of a probed tissue volume, which is derived from said transmitted light, wherein said therapy parameters are obtained from said parameter measurements, and wherein said optical diagnostic tomographic calculation unit (128) is operative based on Diffuse Optical Tomography (DOT) for obtaining said optical properties from said transmitted light; and said control unit (122) is adapted for a feed-back to control an on-going PDT therapy in said dosimetry unit (105) based on said therapy parameters from said optical diagnostic tomographic calculation unit (128) **characterized by** said Diffuse Optical Tomography retrieve changes in said absorption coefficient by equation

$$\Delta \ln\left(\Phi_m\right) = W\Delta\mu_{am}$$

    where $\Delta\ln(\Phi_m)$ is a difference in measured quantities of said transmitted light, and W is a sensitivity matrix defining to what extent detected intensity for each source-detector pair of said optical fibers are affected by changes in said absorption in a small volume element inside a geometry of tissue volume to be treated.

2.  The PDT system according to claim 1, wherein said diagnostic tomographic calculation unit is adapted to provide said therapy parameters from reconstruction methods based on time-domain or frequency-domain measurements.

3.  The PDT system according to claim 1, wherein said diagnostic tomographic calculation unit is configured to provide an accurate reconstruction of optical properties in tissue based on non-steady-state data.

4.  The PDT system according to claim 1, wherein said diagnostic tomographic calculation unit is configured to provide said therapy parameters from a non-linear iterative reconstruction method.

5.  The PDT system according to any of the previous claims, wherein said diagnostic tomographic calculation unit is

devised to provide said therapy parameters from an organ comprising a tissue to be treated.

6. The PDT system according to claim 5, wherein said organ is an inner organ, such as comprised in the list of prostate, brain, kidney, liver, pancreas, trachea, oesophagus, or an outer organ.

7. The PDT system according to any of the previous claims, comprising a PDT online dosimetry instrumentation, wherein said PDT system comprises light sources that are arranged to be modulated over time, or a unit to modulate the light emitted from steady state light sources.

8. The PDT system according to claim 7, comprising a unit to resolve frequency and phase for detection of the light in the frequency domain or to resolve the changes in the light signal over time in the time domain.

**Patentansprüche**

1. Fotodynamisches Therapie(PDT)-System, mindestens eine Lichtquelle, Lichtleitfasern zur interstitiellen Positionierung in Gewebe sowie eine Dosimetrie-Einheit (105) zur Ausführung von Tomographie während der PDT umfassend; wobei die Dosimetrie-Einheit (105) eine Messfolge-Einheit (120) zur Überwachung von Parametermessungen der Lichtübertragung zwischen den Fasern bei deren sequenzieller Aussendung von Licht umfasst, während die benachbarten Lichtleitfasern verwendet werden, um übertragenes Licht sowie fluoreszierendes Licht zu erkennen, eine Steuereinheit (122) und eine optische diagnostische tomographische Berechnungseinheit (128), wobei die optische diagnostische tomographische Berechnungseinheit (128) für die tomographische Rekonstruktion einer räumlichen Verteilung von Therapieparametern angepasst ist, wozu die Fotosensibilisatorkonzentration, welche von dem fluoreszierenden Licht abgeleitet wird, sowie die optischen Eigenschaften zählen, zu denen der Absorptionskoeffizient eines abgetasteten Gewebevolumens gehört, der von dem übertragenen Licht abgeleitet wird, wobei die Therapieparameter aus den Parametermessungen gewonnen werden, und wobei sich die optische diagnostische tomographische Berechnungseinheit (128) auf der Grundlage von Diffuser Optischer Tomographie (DOT) betreiben lässt, um die optischen Eigenschaften anhand des übertragenen Lichts zu gewinnen; und
die Steuereinheit (122) für eine Rückkopplung angepasst ist, um eine laufende PDT-Therapie in der Dosimetrie-Einheit (105) auf der Grundlage der Therapieparameter von der optischen diagnostischen tomographischen Berechnungseinheit (128) zu steuern,
**dadurch gekennzeichnet, dass** die Diffuser Optische Tomographie Veränderungen des Absorptionskoeffizienten mittels der Gleichung

$$\Delta \mathrm{In}(\Phi_m) = W \Delta \mu_{am}$$

abruft, wobei es sich bei $\Delta\mathrm{In}(\Phi_m)$ um eine Differenz der gemessenen Mengen an übertragenem Licht handelt, und es sich bei W um eine Empfindlichkeitsmatrix handelt, welche definiert, in welchem Maße die erkannte Intensität für jedes Quelle-Detektor-Paar der Lichtleitfasern durch Veränderungen der Absorption in einem kleinen Volumenelement in einer Geometrie des zu behandelnden Gewebevolumens beeinflusst wird.

2. PDT-System gemäß Anspruch 1, wobei die diagnostische tomographische Berechnungseinheit so angepasst ist, dass sie Therapieparameter aus Rekonstruktionsverfahren auf der Grundlage von Zeitbereichs- oder Frequenzbereichsmessungen liefert.

3. PDT-System gemäß Anspruch 1, wobei die diagnostische tomographische Berechnungseinheit so eingerichtet ist, dass sie eine präzise Rekonstruktion der optischen Eigenschaften in Gewebe auf der Grundlage von nicht-stationären Daten liefert.

4. PDT-System gemäß Anspruch 1, wobei die diagnostische tomographische Berechnungseinheit so eingerichtet ist, dass sie die Therapieparameter aus einem nichtlinearen iterativen Rekonstruktionsverfahren liefert.

5. PDT-System gemäß einem beliebigen der vorhergehenden Ansprüche, wobei die diagnostische tomographische Berechnungseinheit so beschaffen ist, dass sie die Therapieparameter von einem Organ liefert, das ein zu behandelndes Gewebe umfasst.

6. PDT-System gemäß Anspruch 5, wobei es sich bei dem Organ um ein inneres Organ handelt, welches in der Liste

enthalten ist, die aus Prostata, Hirn, Niere, Leber, Pankreas, Trachea, Speiseröhre besteht, oder es sich um ein äußeres Organ handelt.

7. PDT-System gemäß einem beliebigen der vorhergehenden Ansprüche, eine PDT-Online-Dosimetrie-Geräteausstattung umfassend, wobei das PDT-System Lichtquellen umfasst, die so eingerichtet sind, dass sie über die Zeit hinweg moduliert werden, oder eine Einheit zur Modulierung des von stationären Lichtquellen ausgestrahlten Lichtes umfasst.

8. PDT-System gemäß Anspruch 7, eine Einheit zur Klärung der Frequenz und der Phase zur Erkennung des Lichts des Frequenzbereiches oder zur Klärung der Veränderungen des Lichtsignals über die Zeit hinweg in dem Zeitbereich umfassend.

## Revendications

1. Système de thérapie photo-dynamique (TPD) comprenant au moins une source lumineuse, des fibres optiques destinées à être positionnées de manière interstitielle dans un tissu, et une unité de dosimétrie (105), destinée à réaliser une tomographie durant ladite TPD ; où ladite unité de dosimétrie (105) comprend une unité de séquence de mesure (120) destinée à surveiller des mesures de paramètre de transmission de lumière entre lesdites fibres lorsqu'elles émettent de la lumière de manière séquentielle tandis que des fibres optiques avoisinantes sont utilisées pour détecter la lumière transmise ainsi que la lumière de fluorescence, une unité de commande (122) et une unité de calcul tomographique de diagnostic optique (128), où ladite unité de calcul tomographique de diagnostic optique (128) est conçue pour une reconstruction tomographique d'une distribution spatiale de paramètres de thérapie incluant une concentration de photo sensibilisateur, laquelle est obtenue à partir de ladite lumière de fluorescence, et une reconstruction tomographique de propriétés optiques incluant un coefficient d'absorption d'un volume tissulaire sondé, laquelle est obtenue à partir de ladite lumière transmise, où lesdits paramètres de thérapie sont obtenus à partir desdites mesures de paramètre, et où ladite unité de calcul tomographique de diagnostic optique (128) est mise en oeuvre sur la base d'une tomographie optique diffuse (TOD) destinée à obtenir lesdites propriétés optiques à partir de ladite lumière transmise ; et
ladite unité de commande (122) est conçue pour réaliser une rétroaction pour commander une thérapie TPD en cours dans ladite unité de dosimétrie (105) sur la base desdits paramètres de thérapie fournis par ladite unité de calcul tomographique de diagnostic optique (128)
**caractérisé par le fait que** ladite tomographie optique diffuse (TOD) recueille les évolutions dudit coefficient d'ab-sorption par l'équation

$$\Delta\ln(\Phi_m) = W\Delta\mu_{am}$$

où $\Delta\ln(\Phi_m)$ est une différence dans les quantités mesurées de ladite lumière transmise, et W est une matrice de sensibilité définissant dans quelle mesure les intensités détectées pour chaque paire de source-détecteur desdites fibres optiques sont influencées par les évolutions de ladite absorption dans un élément de petit volume à l'intérieur d'une géométrie de volume tissulaire à traiter.

2. Système de TPD selon la revendication 1, dans lequel ladite unité de calcul tomographique de diagnostic est conçue pour fournir lesdits paramètres de thérapie à partir de méthodes de reconstruction basées sur des mesures de domaine fréquentiel ou de domaine temporel.

3. Système de TPD selon la revendication 1, dans lequel ladite unité de calcul tomographique de diagnostic est configurée pour fournir une reconstruction précise de propriétés optiques dans un tissu sur la base de données de régime non permanent.

4. Système de TPD selon la revendication 1, dans lequel ladite unité de calcul tomographique de diagnostic est configurée pour fournir lesdits paramètres de thérapie à partir d'une méthode de reconstruction itérative non linéaire.

5. Système de TPD selon l'une quelconque des revendications précédentes, dans lequel ladite unité de calcul tomographique de diagnostic est conçue pour fournir lesdits paramètres de thérapie à partir d'un organe comprenant un tissu à traiter.

**6.** Système de TPD selon la revendication 5, dans lequel ledit organe est un organe interne, par exemple un organe parmi la liste constituée de la prostate, du cerveau, du rein, du foie, du pancréas, de la trachée, de l'oesophage, ou un organe externe.

**7.** Système de TPD selon l'une quelconque des revendications précédentes, comprenant une instrumentation de dosimétrie en ligne de TPD, où ledit système de TPD comprend des sources lumineuses qui sont agencées de sorte à être modulées dans le temps, ou une unité permettant de moduler la lumière émise par des sources lumineuses en régime permanent.

**8.** Système de TPD selon la revendication 7, comprenant une unité permettant de déterminer par calcul une fréquence et une phase pour une détection de la lumière dans le domaine fréquentiel ou pour déterminer par calcul les évolutions du signal lumineux dans le temps dans le domaine temporel.

**Fig. 1**

**Fig. 2**

**Fig. 3**

**Fig. 4**

## REFERENCES CITED IN THE DESCRIPTION

**Patent documents cited in the description**

- WO 2003041575 A

- WO 2008020050 A

**Non-patent literature cited in the description**

- **DOUGHERTY TJ.** Photodynamic therapy. *Journal of the National Cancer Institute,* 1998, vol. 90, 889-905

- **A. JOHANSSON.** *Journal of Biomedical Optics,* 2006, vol. 11 (3
- **BROOKSBY.** *Journal of Biomedical Optics,* 2005, vol. 10 (5